(19) Europäisches Patentamt
European Patent Office
Office européen des brevets

(11) Publication number: **0 436 183 B1**

# (12) EUROPEAN PATENT SPECIFICATION

(45) Date of publication of patent specification: **15.03.95**

(51) Int. Cl.[6]: **C07C 245/04**

(21) Application number: **90124725.4**

(22) Date of filing: **19.12.90**

## (54) Process for the preparation of symmetrical azodinitrile dicarboxylic acids from keto acids.

(30) Priority: **26.12.89 US 456901**
**26.12.89 US 456886**
**26.12.89 US 457058**

(43) Date of publication of application:
**10.07.91 Bulletin 91/28**

(45) Publication of the grant of the patent:
**15.03.95 Bulletin 95/11**

(84) Designated Contracting States:
**AT BE CH DE DK ES FR GB GR IT LI LU NL SE**

(56) References cited:
**FR-A- 2 578 249**
**US-A- 2 520 338**
**US-A- 3 783 148**
**US-A- 4 028 345**
**US-A- 4 272 435**
**US-A- 4 684 717**
**US-A- 4 684 718**

(73) Proprietor: **The B.F. Goodrich Company**
**3925 Embassy Parkway**
**Akron**
**Ohio 44313 (US)**

(72) Inventor: **Lai, John Ta-yuan**
**3465 Ridge Park Boulevard**
**Broadview Heights,**
**Ohio 44147 (US)**

(74) Representative: **von Kreisler, Alek,**
**Dipl.-Chem. et al**
**Patentanwälte**
**von Kreisler-Selting-Werner**
**Postfach 10 22 41**
**D-50462 Köln (DE)**

EP 0 436 183 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

## Description

The present invention relates to an improved process for the preparation of symmetrical azo initiators from keto acids utilizing a metal hypochlorite.

## BACKGROUND

It is known in the art to prepare azo compounds from a tertiary amine.

U.S.-A-3,783,148 relates to the preparation of symmetrical azo compounds by the following reaction:

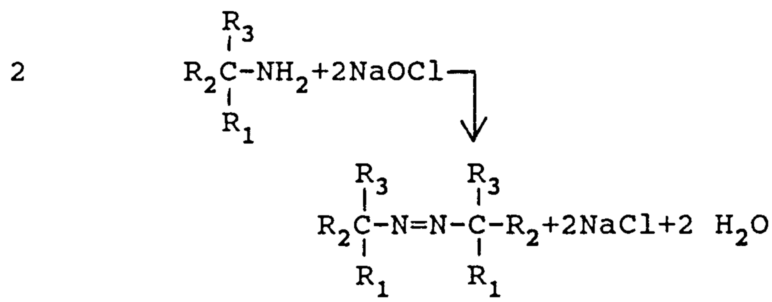

In the above reaction $R_1$ is alkyl of from 1 to 6 carbon atoms, optionally substituted with an alkoxy group of 1 to 4 carbon atoms; $R_2$ is cycloalkyl of from 3 to 6 carbon atoms or alkyl of from 1 to 6 carbon atoms; $R_3$ is a radical selected from the group consisting of CN, COOR, and COOM, where R is an alkyl radical of from 1 to 6 carbon atoms, and M is sodium or potassium; with the proviso that $R_1$ and $R_2$ together contain a total of at least 4 carbon atoms, and with the further proviso that $R_1$ and $R_2$ can be taken together and are alkylene of from 3 to 11 carbon atoms. The coupling reaction occurs in the presence of at least 95 percent by volume of a $C_1$ - $C_2$ alcohol.

U.S.-A-4,028,345 relates to an improved process for the preparation of aliphatic azo compounds.

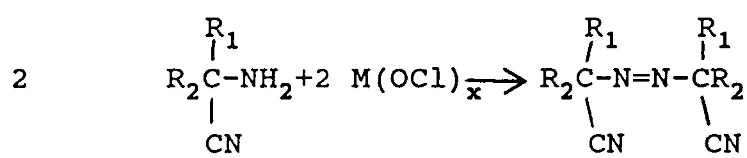

wherein $R_1$ and $R_2$ are selected from the group consisting of aliphatic hydrocarbon radicals of 1 to 8 carbons, optionally substituted with a carboxyl group, a hydroxyl group or an alkoxy group represented by -OR wherein R is an acrylic aliphatic hydrocarbon radical of 1 to 4 carbon atoms; x is the valence of the M ion and a surface active compound selected from the group consisting of an anionic, cationic, nonionic, amphoteric and mixed surface active agents or surfactants. More specifically, this reference relates to a process for the preparation of aliphatic azodinitrile compounds by reacting an aqueous hypochlorite solution with an aminonitrile in the presence of a surface active agent.

The above aminonitriles utilized for the preparation of the azo compounds are prepared in a pressure vessel by reacting a ketone with ammonia gas. The vessel is cooled in a dry ice acetone bath. Hydrogen cyanide is then introduced in portions in an amount equivalent to that of the ketone. The reaction vessel is warmed to room temperature and pressurized to 446 kPa (50 psig) with ammonia, heated to 40 °C and 446 kPa (50 psig) for 8 hours and cooled to yield an aminonitrile according to the equation below:

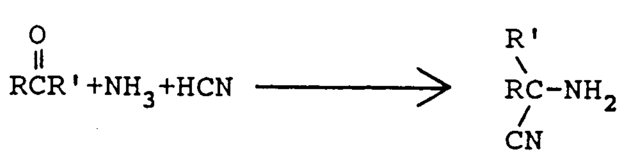

The above references teach the preparation of symmetrical and non-symmetrical azo compounds. In both references, fairly pure aminonitrile is utilized as a starting material for reaction with the metal hypochlorite

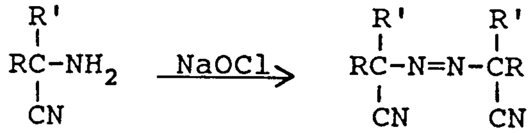

In the past it was thought that an excess of ammonia was to be avoided in the preparation of the aminonitrile since it might interfere with the coupling reaction:

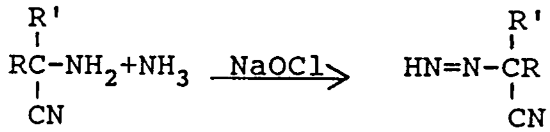


Additionally, water was to be avoided as a solvent in the preparation of the aminonitrile since the water might promote undesirable reverse reactions:

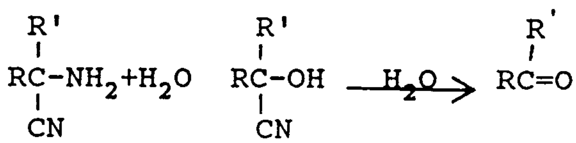


Within the present invention the aminonitrile is formed utilizing a solvent; however, the aminonitrile is not recovered.

U.S.-A-4,684,717 and 4,684,718 provide a process for the preparation of diazocyano acid, which comprises reacting a keto acid or its sodium salt with a cyanogen compound such as sodium cyanide or hydrogen cyanide and a hydrazine in water to form a concentrated aqueous solution of a hydrazo compound, adding acetone and/or water to the concentrated aqueous solution to form a solution of the hydrazo compound, adding chlorine gas to the solution to oxidize the hydrazo compound and form a diazocyano acid, and separating the diazocyano acid from the obtained reaction mixture.

U.S.-A-4,831,096 relates to mixtures of azoalkanes of varying thermal stabilities, at least one of which is an unsymmetrical azoalkane (R-N = N-R'). These unsymmetrical azoalkanes are prepared by reacting four equivalents of a mixture of two or more primary alkyl, cycloalkyl or aralkyl amines with one equivalent of sulfuryl chloride in an inert solvent and oxidizing the resulting mixture of sulfamide products with basic bleach. The unsymmetrical azoalkanes can be separate from the symmetrical azoalkanes by a variety of conventional techniques. The azoalkane mixtures are polymerization initiators for vinyl monomers and curing agents for unsaturated polyester resins.

## SUMMARY OF THE INVENTION

This invention relates to a process for the preparation of a symmetrical azo compound from a keto acid of the formula

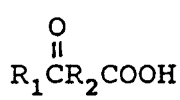

wherein $R_1$ is an alkyl group containing from 1 to 12 carbon atoms and $R_2$ is a direct bond or an alkylene group containing from 1 to 12 carbon atoms. One mole of the keto acid is reacted with from 1 to 2 equivalents of $M(CN)_x$ in an aqueous medium wherein M is a metal comprising lithium, sodium, potassium, magnesium, or calcium and x is the valence of M and from 1 to 2 moles of an ammonia source of ammonium hydroxide (28-30 percent) to form an aqueous solution of an aminonitrile metal carboxylate of the formula

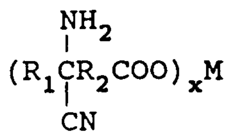

.

$M(CN)_x$ is not volatile and therefore has none of the deleterious properties of HCN which is used in the commercial production of these initiators.

It is surprising that an aqueous solution that contains excess ammonium hydroxide can be used to give a good yield of an azodinitrile without serious interference with the coupling reaction.

Alternatively, the aminonitrile metal carboxylate can be formed by reacting the keto acid, metal cyanide and ammonia using a lower alcohol as a solvent followed by removal of the alcohol under vacuum. The aminonitrile metal carboxylate so formed, instead of the carboxylic acid as claimed in previous references, is reacted with from 1.0 to 2.5 equivalents of $M_1(OCl)_x$ per mole of keto acid with the proviso that no alcohol or surface active agent is utilized, wherein $M_1$ is a metal comprising sodium, potassium, or calcium to form an azodinitrile metal carboxylate of the following structure:

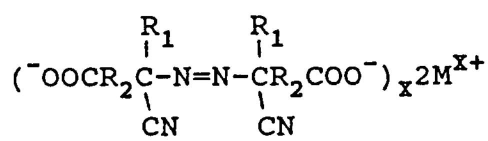

when M is lithium, sodium, potassium, magnesium or calcium and X is the valence of M. The excess $M_1$-$(OCl)_x$ is reduced with a reducing agent comprising sodium bisulfite or sodium sulfite and the metal azo carboxylate is neutralized to form an azodinitrile compound of the formula

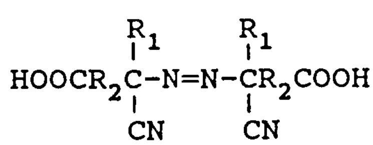

The Keto Acid

The keto acids having utility in this invention are of the general formula

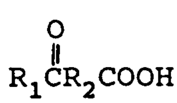

$R_1$ is an alkyl group containing from 1 to 12 carbon atoms, preferably from 1 to 6 carbon atoms and most preferably from 1 to 3 carbon atoms. Such groups are known to those skilled in the art. Examples include methyl, ethyl, propyl, isopropyl, butyl, isobutyl and t-butyl.

$R_2$ is a direct bond or an alkylene group containing from 1 to 12 carbon atoms and preferably 1 to 6 carbon atoms or a cycloalkylene group containing from 3 to 12 carbon atoms. When $R_2$ is not cyclic, $R_2$ most preferably contains from 1 to 4 carbon atoms. When $R_2$ is cyclic it most preferably contains from 3 to 6 carbon atoms. Some examples of $R_2$ cyclic alkylenes are

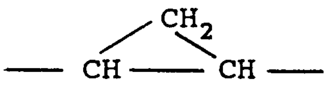 , 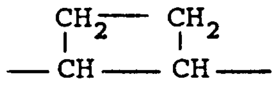 , 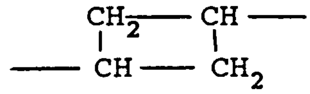 ,

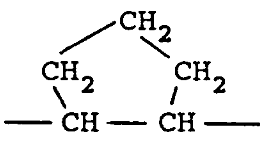 , 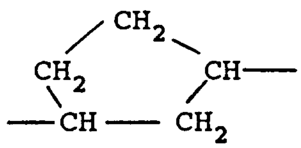 , 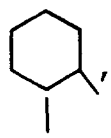 , 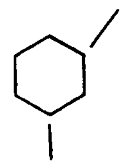 and 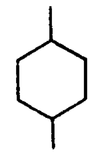

When $R_2$ is not cyclic, examples are methylene, ethylene, propylene, butylene, as well as any branching thereof. The following table lists a few of the many keto acids having utility in this invention. This list is merely illustrative and is not meant to be all-inclusive. A preferred keto acid is levulinic acid.

TABLE I

Keto Acids

| $R_1$ | $R_2$ | Name |
|---|---|---|
| $CH_3$ | direct bond | pyruvic acid |
| $CH_3$ | $CH_2$ | 3-oxobutanoic acid |
| $CH_3$ | $CH_2CH_2$ | levulinic acid |
| $CH_3$ | $CH_2CH_2CH_2$ | 5-oxohexanoic acid |
| $CH_3$ | $CH_2CH_2CH_2CH_2$ | 6-oxoheptanoic acid |
| $CH_3$ | $CH_2CH_2CH_2CH_2CH_2$ | 7-oxooctanoic acid |
| $CH_3CH_2$ | direct bond | 2-oxobutanoic acid |
| $CH_3CH_2$ | $CH_2$ | 3-oxopentanoic acid |
| $CH_3CH_2$ | $CH_2CH_2$ | 4-oxohexanoic acid |
| $CH_3CH_2$ | $CH_2CH_2CH_2$ | 5-oxoheptanoic acid |
| $CH_3CH_2CH_2$ | $CH_2CH_2$ | 4-oxoheptanoic acid |
| $CH_3$ | $CH_2CH(CH_3)$ | 2-methyllevulinic acid |

<u>The Metal Cyanide</u>

One mole of the keto acid is reacted with from 1 to 2, preferably 1 to 1.5, and most preferably from 1 to 1.1 equivalents of a metal cyanide of the formula $M(CN)_x$ wherein the metal M comprises lithium, sodium, potassium, magnesium, or calcium and x is the valence of M. An equivalent of $M(CN)_x$ is its weight in grams, pounds, etc. divided by its equivalent weight. The equivalent weight of $M(CN)_x$ is equal to its molecular weight divided by the valence of x. An equivalent weight of NaCN is 49 (49 molecular weight divided by valence of 1) and 49 grams is one gram-equivalent of NaCN. An equivalent weight of $Ca(CN)_2$ is 46 (92 molecular weight divided by valence of 2) and 46 grams is one gram-equivalent of $Ca(CN)_2$. The reaction of the keto acid with $M(CN)_x$ is a reversible addition reaction that strongly favors the right side with very little by-products formed. A preferred metal cyanide is sodium cyanide.

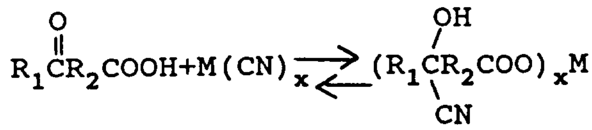

In order to convert the cyanohydrin metal carboxylate to an aminonitrile metal carboxylate, 1 to 2 moles of an of an ammonia source per 0.5-1 moles of keto acid is introduced.

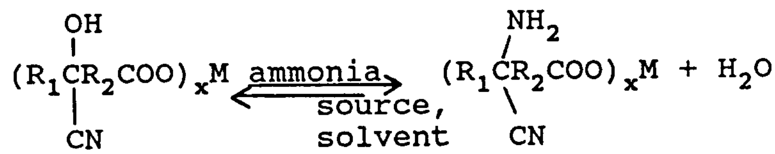


When the ammonia source is ammonium hydroxide (28-30 percent), water is used as a solvent; when the ammonia source is ammonia gas, a lower carbon alcohol is used.

The Metal Hypochlorite

The aminonitrile metal carboxylate is converted to the azodinitrile metal carboxylate by action of $M_1(OCl)_x$ in the ratio of from 1.0 to 2.5, preferably from 1.2 to 2.0, and most preferably from 1.2 to 1.5 equivalents of metal hypochlorite per mole of keto acid. $M_1$ comprises sodium, potassium, or calcium and x is the valence of the metal $M_1$. Sodium is the preferred metal.

Reducing Agent

After the azo compound is formed, the excess unreacted $M_1(OCl)_x$ is removed for environmental concerns. This is done by utilizing a reducing agent such as sodium bisulfite or sodium sulfite. The reducing agent is added as a 20 percent by weight aqueous solution. Further, no alcohol or surface active agent is utilized. Just enough reducing agent is added to provide a negative test for KI-starch test paper; i.e., KI-starch paper is no longer darkened by $I_2$.

$$M_1(OCl)_x + NaHSO_3 \longrightarrow M_1(Cl)_x + NaHSO_4$$

or

$$M_1(OCl)_x + Na_2SO_3 \longrightarrow M_1(Cl)_x + Na_2SO_4$$

Neutralization Agent

Once the metal hypochlorite is reduced, the azodinitrile metal carboxylate is ready to be neutralized to form the azo dicyanocarboxylic acid. The neutralization agent is usually an inorganic acid such as hydrochloric acid, nitric acid or sulfuric acid. The preferred mineral acid is hydrochloric acid. The azodinitrile metal carboxylate is of the following structure

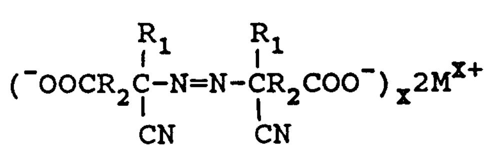

where M is lithium, sodium, potassium, magnesium, or calcium and X is the valence of M. Acidifying the above azodinitrile metal carboxylate yields the azodinitrile of the structure:

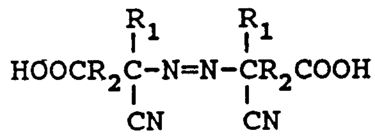

The compositions prepared by the process of this invention are formed by preparing a mixture of metal cyanide, ammonia source and solvent. To this mixture is added a keto acid to form the aminonitrile metal carboxylate,

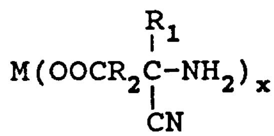

The order of addition can be varied. It may be carried out as above or the metal cyanide can be suspended or dissolved in solvent, the keto acid added to generate a cyanohydrin followed by the addition of the ammonia source.

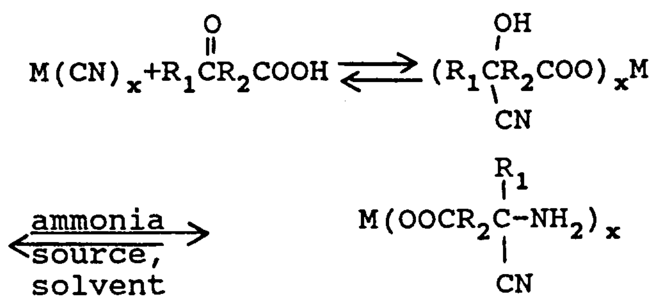


Or the ammonium salt of the acid can be formed first followed by the addition of metal cyanide.

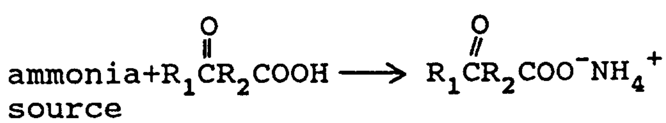


then

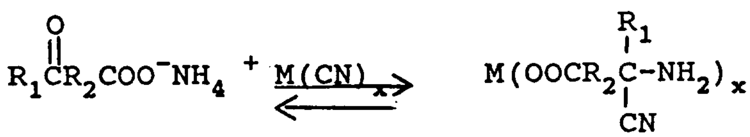

The preferred method is to mix metal cyanide and ammonium hydroxide in water followed by additions of the keto acid. The advantages are (a) the pH is always greater than 8, there is no danger of HCN escaping and (b) all intermediates remain in solutions therefore, no solidification can occur.

If excess ammonia is present after the formation of the aminonitrile metal carboxylate, the overall yield of the azodinitrile metal carboxylate is not diminished but rather more metal hypochlorite will have to be used to convert the amino metal carboxylate to the azodinitrile metal carboxylate. The solvent can be water or a lower carbon alcohol such as methanol or ethanol, preferably methanol. If a lower carbon alcohol is used as the solvent, it has to be removed and when this is done, the aminonitrile metal carboxylate is obtained as an oil which tends to solidify. If the lower carbon alcohol is not removed before the addition of the metal hypochlorite, lower yields of azodinitriles are obtained. An advantage of utilizing water as the solvent is that the processing is easier due to the fact that the aminonitrile metal carboxylate does not have to be isolated.

The molar ratio of keto acid:ammonia source is from 0.5-1:1-2, preferably from 1:1-1.5 and most preferably 1:1-1.1. After reaction of the ammonia source, nitrogen is bubbled through the reaction mixture to

remove excess ammonia source. The equivalent ratio of amino nitrile metal carboxylate: $M_1(OCl)_x$ is from 1:1.0-2.75 preferably 1:1.3-2.0 and most preferably 1:1.5-1.8. The excess $M_1(OCl)_x$ is neutralized with up to a 100 percent excess of $NaHSO_3$ or $Na_2SO_3$,preferably a 0-10 percent excess and most preferably a 0-2 percent excess of reducing agent. The reaction temperature for any of the intermediates is from 0°C to 60°C preferably from 25 to 50°C and most preferably from 30 to 40°C. The reaction temperature for the coupling of aminonitrile metal carboxylate to form the azodinitrile metal carboxylate is from -15 to 35°C, preferably from 0 to 25°C and most preferably from 0 to 10°C.

The azodinitrile produced by the process of this invention can be used as a polymerization initiator in emulsion, dispersion and solution polymerization systems. Polymerization involving vinyl chloride, methyl methacrylate, and butadiene-styrene are merely examples of such systems in industry that would benefit from the use of such initiators.

If an azodinitrile containing a low salt content is desired, the azodinitrile can be washed with water either before or after isolation.

The following examples are illustrative of the preparation of azodinitriles. Unless otherwise indicated, all parts and percentages are by weight.

EXAMPLE 1

Charged to a 50 milliliter, three-neck round bottom flask fitted with a thermometer and magnetic stirrer was 5.15 parts (0.105 moles) sodium cyanide, 6 parts water and 6.4 parts (0.105 moles) 28 percent ammonium hydroxide in water. The contents were stirred and cooled in a water bath. At 25°C 11.6 parts (0.1 mole) levulinic acid dissolved in 7 parts water was added drop-wise. After completion of the levulinic acid addition, the water bath was removed and the temperature was raised to 35°C.

To a 500 milliliter jacketed flask with a thermometer and mechanical stirrer was added 130 parts of a 13 percent aqueous solution of sodium hypochlorite and the temperature was lowered to below minus 5°C. The contents of the 50 milliliter flask were added dropwise over two hours at a temperature range of between minus 9°C to minus 5°C. After the addition was complete, 30 parts of a 19 percent aqueous solution of sodium bisulfite were added to reduce the excess sodium hypochlorite. The pH was 10. An additional 3.2 parts sodium bisulfite solution was added and the resulting pH was 7. Ten parts concentrated hydrochloric acid were added to convert the sodium salt to the free carboxylic acid. The pH was 2. The contents were filtered and the solid product water-washed to obtain 11.2 parts of a white solid having the following structure:

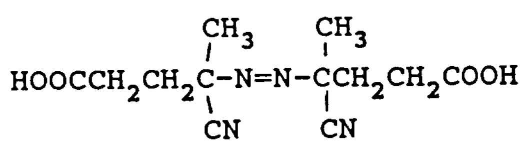

EXAMPLE 2

Added to a one liter three-neck round bottom flask with thermometer and mechanical stirrer was 116 parts (1 mole) levulinic acid and 400 parts methanol. The contents were cooled to 20°C and 51.5 parts (1.05 moles) sodium cyanide were added in portions. After all the sodium cyanide was added, ammonia gas was bubbled below the surface over a two hour period while maintaining the temperature at 20°C. The contents were stirred at 25°C for three hours and filtered and the solid product was rinsed with isopropyl alcohol. 150.1 parts of a sodium salt of an amino nitrile was obtained.

To a two liter three-neck round bottom flask with thermometer and mechanical stirrer is added 860 parts of a 13 percent aqueous solution of sodium hypochlorite and the temperature is lowered to below minus 5°C. About 150 parts of the sodium salt of the amino nitrile prepared above is dissolved in 300 parts water and are added drop-wise over a three hour period while maintaining the temperature at between minus 10° to minus 5°C. After this addition is complete, 290 parts of a 19 percent aqueous solution of sodium bisulfite is added to reduce the excess sodium hypochlorite. About 500 parts concentrated HCl is added to convert the sodium salt to the free carboxylic acid. The contents are filtered to give a product having the same structure as in Example 1.

EXAMPLE 3

Charged to a one liter three-neck round bottom flask with thermometer and mechanical stirrer are 51.5 parts (1.05 moles) sodium cyanide, 75 parts water and 64 parts (1.05 moles) 28 percent ammonium hydroxide in water. The contents are stirred and cooled in a water bath. At 27 °C, 130 parts (1 mole) 5-oxohexanoic acid is added in a slow stream. After the 5-oxohexanoic acid addition is complete, the water bath is removed and the temperature is raised to 37 °C.

To a two liter jacketed flask with a thermometer and a mechanical stirrer is added 573 parts of a 13 percent aqueous solution of potassium hypochlorite and the temperature is lowered to -5 °C. The contents of the one liter flask are added drop-wise at a temperature range of between -10 to -5 °C. After the addition is complete, 550 parts of a 19 percent aqueous solution of sodium bisulfite are added to reduce the excess sodium hypochlorite. The pH is at about 10. Nine hundred parts concentrated HCl is added to convert the sodium salt to the free carboxylic acid. The contents are filtered to give a product having the following structure:

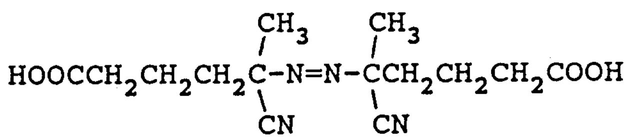

EXAMPLE 4

Charged to a 50-ml, 3-neck, round-bottom flask with thermometer and magnetic stirrer, was 5.15 parts (0.105 moles) sodium cyanide, 17 parts (0.105 moles) 28 percent ammonium hydroxide in water, and 2 parts water. The closed system was cooled in a water bath and at about 35 °C 11.6 parts (0.1 moles) levulinic acid was added drop-wise. The reaction temperature was maintained at between 35-40 °C for 2 hours. The contents were cooled to 25 °C and nitrogen was bubbled below the surface for 1 hour to remove excess ammonia.

To a 300-ml jacketed flask with thermometer and mechanical stirrer was added 74.5 parts (0.14 moles) of the 14.5 percent by weight aqueous solution of sodium hypochlorite and the contents were cooled to about 0 °C. The contents of the 50-ml flask were then added drop-wise at between 0-5 °C over 52 minutes. After the addition was complete, the contents were stirred for 1.5 hours at 0 °C. 5.9 parts of a 20 percent aqueous sodium sulfite solution was added until there was no reaction with KI paper. The pH of the solution was 12. 40 g water was added followed by 12.5 ml concentrated hydrochloric acid and the pH was lowered to about 1. The contents were filtered and rinsed with 30 ml portions of water three times to give a product having the same structure as in Example 1.

## Claims

**1.** A process for the preparation of symmetrical azo compounds, comprising,
reacting a keto acid

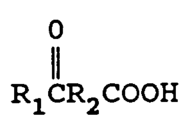

wherein $R_1$ is an alkyl group containing from 1 to 12 carbon atoms, and $R_2$ is a direct bond, or an alkylene group containing from 1 to 12 carbon atoms, or a cycloalkylene group containing from 3 to 12 carbon atoms

with from 1 to 2 equivalents of $M(CN)_x$ per mole of keto acid wherein M is a metal comprising lithium, sodium, potassium, magnesium, or calcium and x is the valence of M and an ammonia source in a molar ratio of keto acid:ammonia source of from 1:1-4 in the presence of a solvent to form an aminonitrile metal carboxylate of the formula

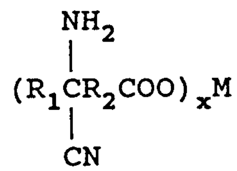

removing excess ammonia source,
removing methanol if used as a solvent, and
reacting said aminonitrile metal carboxylate with from 1.0 to 2.5 equivalents $M_1(OCl)_x$ per mole of keto acid with the proviso that no alcohol or surface active agent is utilized with $M_1(OCl)_x$ wherein $M_1$ is a metal comprising sodium, potassium, or calcium and x is the valence of $M_1$ to form an azodinitrile metal carboxylate comprising

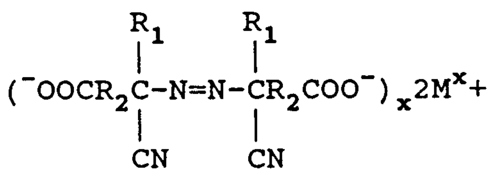

reducing excess $M_1(OCl)_x$ with a reducing agent, and further
neutralizing the azodinitrile metal carboxylate to form

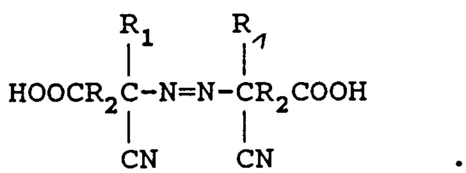

2. The process of Claim 1, wherein $R_1$ is an alkyl group containing from 1 to 6 carbon atoms, and $R_2$ is an alkylene group containing from 1 to 6 carbon atoms, or a cycloalkylene group containing from 3 to 6 carbon atoms, wherein the molar ratio of said keto acid to said ammonia source is 1:1-1.5, wherein the molar ratio of said metal cyanide to said keto acid is 1-1.5:1, and wherein the molar ratio of said metal hypochlorite to said keto acid is 1:1.2 to 2.0.

3. The process of any preceding claim, wherein the reducing agent is sodium bisulfite or sodium sulfite in an amount up to 100 percent excess, wherein the azodinitrile metal carboxylate is neutralized with a mineral acid comprising nitric acid, sulfuric acid, or hydrochloric acid, wherein said solvent is water or methanol, and wherein said ammonia source is ammonia or ammonia hydroxide.

4. A process for the preparation of symmetrical azo compounds, comprising,
reacting a keto acid

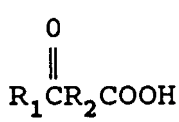

wherein $R_1$ is an alkyl group containing from 1 to 12 carbon atoms, and $R_2$ is a direct bond, or an alkylene group containing from 1 to 12 carbon atoms with from 1 to 2 equivalents of $M(CN)_x$ per mole of keto acid, wherein M is a metal comprising lithium, sodium, potassium, magnesium, or calcium and x is the valence of M to form a cyanohydrin metal carboxylate of the formula

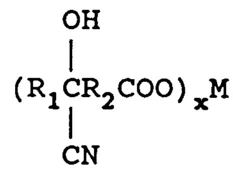

and

reacting said cyanohydrin metal carboxylate with an ammonia source in a molar ratio of keto acid:ammonia source of from 1:1-4 in the presence of a solvent to form an aminonitrile metal carboxylate of the formula

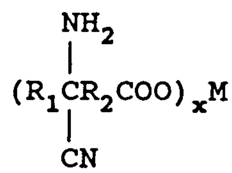

removing excess ammonia source,

reacting said aminonitrile metal carboxylate with from 1.0 to 2.5 equivalents $M_1(OCl)_x$ per mole of keto acid with the proviso that no alcohol or surface active agent is utilized wherein $M_1$ is a metal comprising sodium, potassium, or calcium and x is the valence of $M_1$ to form an azodinitrile metal carboxylate comprising

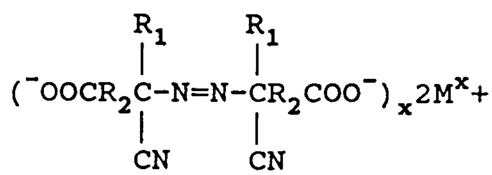

reducing excess $M_1(OCl)_x$ with a reducing agent, and further neutralizing the azodinitrile metal carboxylate to form

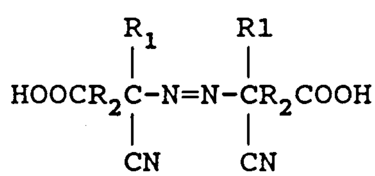

.

5. The process of Claim 4, wherein $R_1$ is a methyl group and $R_2$ is an ethyl group, wherein M equals sodium, and wherein the ammonia source is ammonium hydroxide, and the solvent is water.

6. The process of any preceding claim, wherein the reducing agent is in an amount from 0 to 10 percent excess, wherein the neutralization agent is hydrochloric acid, wherein the temperature of the reaction for the intermediates is from 30°C to 40°C, and the reaction temperature for the coupling reactions is from 0°C to 10°C.

7. The process of any preceding claim, wherein $R_1$ is an alkyl group containing from 1 to 6 carbon atoms, and $R_2$ is an alkylene group containing from 1 to 6 carbon atoms, or a cycloalkylene group containing from 3 to 6 carbon atoms, wherein the molar ratio of said keto acid to said ammonia source is 1:1-1.5, wherein the molar ratio of said metal cyanide to said keto acid is 1-1.5:1, wherein the molar ratio of said metal hypochlorite to said keto acid is 1:1.2 to 2.0; and

wherein the reducing agent is sodium bisulfite or sodium sulfite in an amount up to 100 percent excess.

8. The process of any preceding claim, wherein the azodinitrile metal carboxylate is neutralized with a mineral acid comprising nitric acid, sulfuric acid, or hydrochloric acid, wherein said solvent is water or methanol, and wherein said ammonia source is ammonia or ammonia hydroxide; and
   wherein $R_1$ is a methyl group and $R_2$ is an ethyl group.

9. The process of any preceding claim, wherein M equals sodium, and wherein the ammonia source is ammonium hydroxide, and the solvent is water, wherein the reducing agent is in an amount from 0 to 10 percent excess;
   wherein the neutralization agent is hydrochloric acid; and
   wherein the temperature of the reaction for the intermediates is from 30 ° C to 40 ° C, and the reaction temperature for the coupling reactions is from 0 ° C to 10 ° C.

**Patentansprüche**

1. Verfahren zur Herstellung symmetrischer Azoverbindungen, umfassend:
   Umsetzen einer Ketosäure

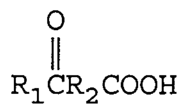

worin $R_1$ eine Alkylgruppe mit 1 bis 12 Kohlenstoffatomen ist und $R_2$ eine direkte Bindung oder eine Alkylengruppe mit 1 bis 12 Kohlenstoffatomen oder eine Cycloalkylengruppe mit 3 bis 12 Kohlenstoffatomen ist,
mit von 1 bis 2 Äquivalenten $M(CN)_x$ pro Mol Ketosäure, worin M eines der Metalle Lithium, Natrium, Kalium, Magnesium oder Calcium ist und x die Wertigkeit von M ist, und einer Ammoniakquelle in einem molaren Verhältnis von Ketosäure: Ammoniakquelle von 1:1-4 in Gegenwart eines Lösungsmittels unter Bildung eines Metallaminonitrilcarboxylats der Formel

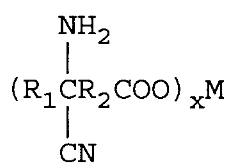

Entfernen überschüssiger Ammoniakquelle,
Entfernen des Methanols, falls dieses als Lösungsmittel verwendet wurde, sowie
Umsetzen des Metallaminonitrilcarboxylats mit von 1,0 bis 2,5 Äquivalenten $M_1(OCl)_x$ pro Mol Ketosäure, mit der Maßgabe, daß mit $M_1(OCl)_x$ kein Alkohol oder Tensid eingesetzt wird, wobei $M_1$ eines der Metalle Natrium, Kalium oder Calcium ist und x die Wertigkeit von $M_1$ ist, unter Bildung eines Metallazodinitrilcarboxylats, umfassend

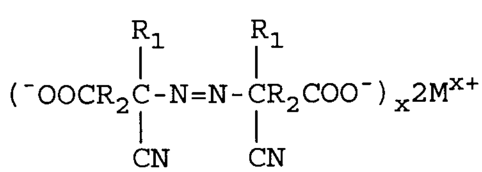

Reduzieren von überschüssigem $M_1(OCl)_x$ mit einem Reduktionsmittel sowie weiterhin
Neutralisieren des Metallazodinitrilcarboxylats unter Bildung von

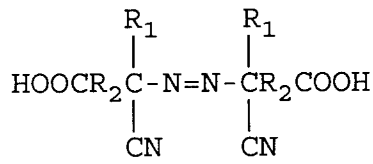 .

2. Verfahren gemäß Anspruch 1, wobei $R_1$ eine Alkylgruppe mit 1 bis 6 Kohlenstoffatomen ist und $R_2$ eine Alkylengruppe mit 1 bis 6 Kohlenstoffatomen oder eine Cycloalkylengruppe mit 3 bis 6 Kohlenstoffatomen ist, wobei das Stoffmengenverhältnis der Ketosäure zu der Ammoniakquelle 1:1-1,5 beträgt, wobei das Stoffmengenverhältnis des Metallcyanids zu der Ketosäure 1-1,5:1 beträgt und wobei das Stoffmengenverhältnis des Metallhypochlorits zu der Ketosäure 1:1,2 bis 2,0 beträgt.

3. Verfahren gemäß einem der vorangehenden Ansprüche, wobei das Reduktionsmittel Natriumhydrogensulfit oder Natriumsulfit in einer Menge von bis zu 100 Prozent Überschuß ist, wobei das Metallazodinitrilcarboxylat mit einer der Mineralsäuren Salpetersäure, Schwefelsäure oder Salzsäure neutralisiert wird, wobei das Lösungsmittel Wasser oder Methanol ist und wobei die Ammoniakquelle Ammoniak oder Ammoniakwasser ist.

4. Verfahren zur Herstellung symmetrischer Azoverbindungen, umfassend:
Umsetzen einer Ketosäure

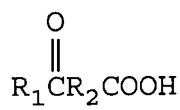

worin $R_1$ eine Alkylgruppe mit 1 bis 12 Kohlenstoffatomen ist und $R_2$ eine direkte Bindung oder eine Alkylengruppe mit 1 bis 12 Kohlenstoffatomen ist mit von 1 bis 2 Äquivalenten $M(CN)_x$ pro Mol Ketosäure, worin M eines der Metalle Lithium, Natrium, Kalium, Magnesium oder Calcium ist und x die Wertigkeit von M ist, unter Bildung eines Metallcyanhydrincarboxylats der Formel

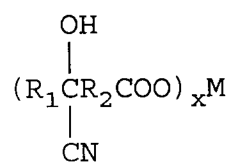

und Umsetzen des Metallcyanhydrincarboxylats mit einer Ammoniakquelle in einem molaren Verhältnis von Ketosäure: Ammoniakquelle von 1:1-4 in Gegenwart eines Lösungsmittels unter Bildung eines Metallaminonitrilcarboxylats der Formel

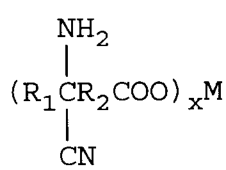

Entfernen überschüssiger Ammoniakquelle,
Umsetzen des Metallaminonitrilcarboxylats mit von 1,0 bis 2,5 Äquivalenten $M_1(OCl)_x$ pro Mol Ketosäure, mit der Maßgabe, daß kein Alkohol oder Tensid eingesetzt wird, wobei $M_1$ eines der Metalle Natrium, Kalium oder Calcium ist und x die Wertigkeit von $M_1$ ist, unter Bildung eines Metallazodinitrilcarboxylats, umfassend

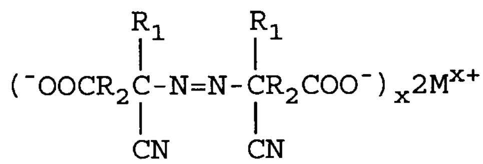

Reduzieren von überschüssigem $M_1(OCl)_x$ mit einem Reduktionsmittel sowie weiterhin Neutralisieren des Metallazodinitrilcarboxylats unter Bildung von

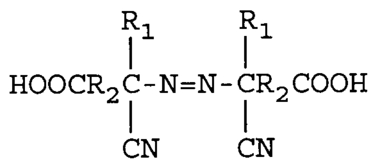
.

5. Verfahren gemäß Anspruch 4, wobei $R_1$ eine Methylgruppe ist und $R_2$ eine Ethylgruppe ist, wobei M gleich Natrium ist und wobei die Ammoniakquelle Ammoniakwasser ist und das Lösungsmittel Wasser ist.

6. Verfahren gemäß einem der vorangehenden Ansprüche, wobei das Reduktionsmittel in einer Menge von 0 bis 10 Prozent Überschuß eingesetzt wird, wobei das Neutralisationsmittel Salzsäure ist, wobei die Reaktionstemperatur für die Zwischenprodukte von 30 ° C bis 40 ° C beträgt und die Reaktionstemperatur für die Kopplungsreaktionen von 0 ° C bis 10 ° C beträgt.

7. Verfahren gemäß einem der vorangehenden Ansprüche, wobei $R_1$ eine Alkylgruppe mit 1 bis 6 Kohlenstoffatomen ist und $R_2$ eine Alkylengruppe mit 1 bis 6 Kohlenstoffatomen oder eine Cycloalkylengruppe mit 3 bis 6 Kohlenstoffatomen ist, wobei das Stoffmengenverhältnis der Ketosäure zu der Ammoniakquelle 1:1-1,5 beträgt, wobei das Stoffmengenverhältnis des Metallcyanids zu der Ketosäure 1-1,5:1 beträgt, wobei das Stoffmengenverhältnis des Metallhypochlorits zu der Ketosäure 1:1,2 bis 2,0 beträgt und wobei das Reduktionsmittel Natriumhydrogensulfit oder Natriumsulfit in einer Menge von bis zu 100 Prozent Überschuß ist.

8. Verfahren gemäß einem der vorangehenden Ansprüche, wobei das Metallazodinitrilcarboxylat mit einer der Mineralsäuren Salpetersäure, Schwefelsäure oder Salzsäure neutralisiert wird, wobei das Lösungsmittel Wasser oder Methanol ist und wobei die Ammoniakquelle Ammoniak oder Ammoniakwasser ist und wobei $R_1$ eine Methylgruppe ist und $R_2$ eine Ethylgruppe ist.

9. Verfahren gemäß einem der vorangehenden Ansprüche, wobei M gleich Natrium ist und wobei die Ammoniakquelle Ammoniakwasser ist und das Lösungsmittel Wasser ist, wobei das Reduktionsmittel in einer Menge von 0 bis 10 Prozent Überschuß eingesetzt wird, wobei das Neutralisationsmittel Salzsäure ist und wobei die Reaktionstemperatur für die Zwischenprodukte von 30 ° C bis 40 ° C beträgt und die Reaktionstemperatur für die Kopplungsreaktionen von 0 ° C bis 10 ° C beträgt.

## Revendications

1. Procédé pour préparer des composés azoïques symétriques, qui consiste :
   à faire réagir un oxo-acide

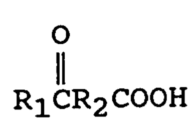

dans laquelle $R_1$ est un groupe alkyle ayant de 1 à 12 atomes de carbone, et $R_2$ est une liaison directe, un groupe alkylène ayant de 1 à 12 atomes de carbone, ou un groupe cycloalkylène ayant de

3 à 12 atomes de carbone, avec 1 à 2 équivalents de $M(CN)_x$ par mole d'oxo-acide, où M est un métal choisi parmi l'ensemble comprenant le lithium, le sodium, le potassium, le magnésium et le calcium, et x est la valence de M, et une source d'ammoniac, selon un rapport en moles de l'oxo-acide à la source d'ammoniac de 1:1-4, en présence d'un solvant, pour former un aminonitrilecarboxylate métallique de formule

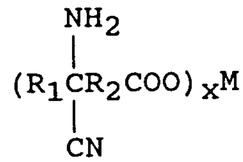

à éliminer la source d'ammoniac en excès,

à éliminer le méthanol s'il est utilisé comme solvant, et

à faire réagir l'aminonitrilecarboxylate métallique avec de 1,0 à 2,5 équivalents de $M_1(OCl)_x$ par mole d'oxo-acide, du moment que l'on n'utilise aucun alcool ou agent tensioactif avec $M_1(OCl)_x$, où $M_1$ est un métal, comprenant le sodium, le potassium ou le calcium, et x est la valence de $M_1$, pour former un azodinitrilecarboxylate métallique de formule

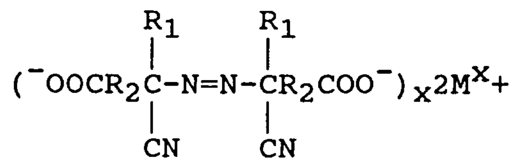

à réduire le $M_1(OCl)_x$ en excès à l'aide d'un réducteur, et en outre

à neutraliser l'azodinitrilecarboxylate métallique pour former le composé suivant

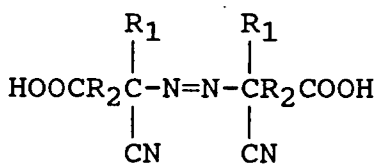

2. Procédé selon la revendication 1, dans lequel $R_1$ est un groupe alkyle ayant de 1 à 6 atomes de carbone, et $R_2$ est un groupe alkylène ayant de 1 à 6 atomes de carbone, ou un groupe cycloalkylène ayant de 3 à 6 atomes de carbone, où le rapport en moles de l'oxo-acide à la source d'ammoniac est de 1:1-1,5, le rapport en moles du cyanure métallique à l'oxo-acide est de 1-1,5:1 et le rapport en moles de l'hypochlorite métallique à l'oxo-acide est de 1:1,2 à 2,0.

3. Procédé selon l'une quelconque des revendications précédentes, dans lequel le réducteur est le bisulfite de sodium ou le sulfite de sodium, en une quantité allant jusqu'à un excès de 100 %, l'azodinitrilecarboxylate métallique étant neutralisé par un acide minéral, comprenant l'acide nitrique, l'acide sulfurique ou l'acide chlorhydrique, le solvant étant l'eau ou le méthanol, et la source d'ammoniac étant l'ammoniac ou l'hydroxyde d'ammonium.

4. Procédé pour préparer des composés azoïques symétriques, qui consiste :

à faire réagir un oxo-acide

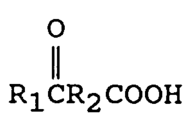

où $R_1$ est un groupe alkyle ayant de 1 à 12 atomes de carbone, et $R_2$ est une liaison directe ou un groupe alkylène ayant de 1 à 12 atomes de carbone, avec de 1 à 2 équivalents de $M(CN)_x$ par mole d'oxo-acide, où M est un métal choisi parmi l'ensemble comprenant le lithium, le sodium, le potassium, le magnésium et le calcium, et x est la valence de M, pour former un cyanohydrinecarboxylate métallique de formule

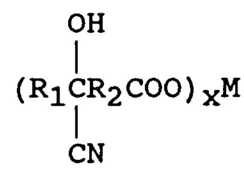

et

à faire réagir le cyanohydrinecarboxylate métallique avec une source d'ammoniac selon un rapport en moles de l'oxo-acide à la source d'ammoniac de 1:1-4, en présence d'un solvant, pour former un aminonitrilecarboxylate métallique de formule

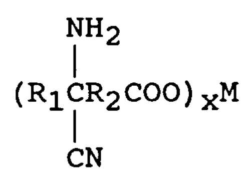

à éliminer la source d'ammoniac en excès,

à faire réagir l'aminonitrilecarboxylate métallique avec de 1,0 à 2,5 équivalents de $M_1(OCl)_x$ par mole d'oxo-acide, du moment que l'on n'utilise aucun alcool ou agent tensioactif, où $M_1$ est un métal comprenant le sodium, le potassium ou le calcium, et x est la valence de $M_1$, pour former un azodinitrilecarboxylate métallique de formule

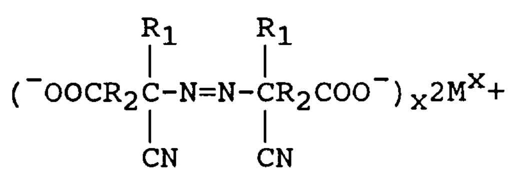

à réduire le $M_1(OCl)_x$ en excès à l'aide d'un réducteur, et en outre

à neutraliser l'azodinitrilecarboxylate métallique pour former le composé suivant :

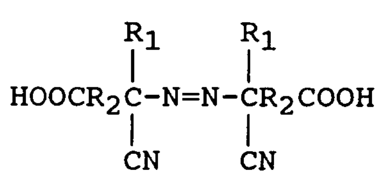

**5.** Procédé selon la revendication 4, dans lequel $R_1$ est un groupe méthyle, et $R_2$ est un groupe éthyle, M étant le sodium, la source d'ammoniac étant l'hydroxyde d'ammonium et le solvant étant l'eau.

**6.** Procédé selon l'une quelconque des revendications précédentes, dans lequel le réducteur est présent en une quantité en excès de 1 à 10 %, l'agent de neutralisation est l'acide chlorhydrique, la température de la réaction est de 30 à 40 °C pour les intermédiaires, et la température de réaction est de 0 à 10 °C pour les réactions de copulation.

7. Procédé selon l'une quelconque des revendications précédentes, dans lequel $R_1$ est un groupe alkyle ayant de 1 à 6 atomes de carbone, et $R_2$ est un groupe alkylène ayant de 1 à 6 atomes de carbone, ou un groupe cycloalkylène ayant de 3 à 6 atomes de carbone, où le rapport en moles de l'oxo-acide à la source d'ammoniac est de 1:1-1,5, le rapport en moles du cyanure métallique à l'oxo-acide est de 1-1,5:1 et le rapport en moles de l'hypochlorite métallique à l'oxoacide est de 1:1,2 à 2,0 ; et
   où le réducteur est le bisulfite de sodium ou le sulfite de sodium en une quantité allant jusqu'à un excès de 100 %.

8. Procédé selon l'une quelconque des revendications précédentes, dans lequel l'azodinitrilecarboxylate métallique est neutralisé par un acide minéral choisi parmi l'ensemble comprenant l'acide nitrique, l'acide sulfurique et l'acide chlorhydrique, le solvant étant l'eau ou le méthanol, la source d'ammoniac étant l'ammoniac ou l'hydroxyde d'ammonium ; et
   où $R_1$ est un groupe méthyle et $R_2$ est un groupe éthyle.

9. Procédé selon l'une quelconque des revendications précédentes, dans lequel M est le sodium, et où la source d'ammoniac est l'hydroxyde d'ammonium, le solvant est l'eau, et le réducteur est présent en une quantité correspondant à un excès de 0 à 10 % ;
   l'agent de neutralisation étant l'acide chlorhydrique ; et
   où la température de la réaction est de 30 à 40 °C pour les intermédiaires, et la température de réaction est de 0 à 10 °C pour les réactions de copulation.